# EUROPEAN PATENT APPLICATION

(11) **EP 2 275 812 A1**
(43) Date of publication of application: **19.01.2011**
(21) Application number: 09009208.1
(22) Date of filing: 15.07.2009
(51) Int. Cl.: G01N 33/34, G01N 33/36, G01N 33/38, B65H 23/188, G01L 5/10

(54) **Apparatus for monitoring mechanical properties of a moving filamentary or planar body and monitoring process**

(71) Applicant: Stichting Dutch Polymer Institute, 5612 AB Eindhoven (NL)
(72) Inventor: The designation of the inventor has not yet been filed
(74) Representative: Ackermann, Joachim

(57) **Abstract**

Apparatus for monitoring mechanical properties of a moving filamentary or planar body and monitoring process

Disclosed is an apparatus and a method for monitoring mechanical properties of a moving filamentary or planar body.

The apparatus is characterized by the presence of the elements:
a) means for causing a movement of the body along a predetermined passage, wherein in at least a part of the passage the body is levitating,
b) means for generating deflection and/or elongation of pre-determined length of the body which is positioned in that part of the passage, wherein the body is levitating, or which is positioned at the beginning or at the end of the predetermined passage, and
c) means for metering a force and/or a change of velocity of the moving body created by the deflection and/or elongation of the body.

The apparatus can be used for on-line monitoring of mechanical properties of filaments or films after compounding and extrusion. The apparatus detects mechanical properties on-line so that the process is not interrupted. Between processing, extrusion and testing no further process step is necessary, like cutting, punching or injection molding. This results in an acceleration of development time in combinatorial development processes and in a simple and reliable technique to monitor process parameters of filament and film production lines on-line.

## Description

This invention relates to an apparatus for monitoring mechanical properties of filaments or films during, for example during a production process.

During the production of filamentary or planar materials the specifications of the materials need to be controlled. There are already online monitoring processes available which are coupled with one or more process parameters in order to optimize said process parameters.

In filament or film production lines, for example, the speed of single rolls transporting and/or deforming the products can be monitored and adjusted in order to obtain a constant drawing ratio. Or the product during its transfer through the production line is monitored by a camera in order to detect the number of defects induced during the process. The result of this inspection may be used to establish a feed back loop in order to control one or more process parameters, such as to control the the speed of selected rolls in the production line.

Alternative monitoring techniques encompass on-line techniques, such as rheometry, different spectroscopy techniques, e.g. FT-IR-, NIR-, or UV-VIS-spectrocopy, light scattering techniques or ultrasonic wave techniques, which can be used to monitor the operation of an extruder or a film production line. Instead thereof or in addition to on-line techniques in-line tests can be performed during the production process. Examples thereof are hardness or penetration tests, use of punch press for generation of off-line test specimens, or ultrasonic waves test. Instead thereof or in addition to on-line techniques and/or to in-line tests off-line tests can be performed using the end product of the process or using specimens obtained during the production of the end product. Examples thereof are the investigation of pelletized or punched specimens from the production process or the investigation of the end product by means of gel-permeation chromatography, high performance liquid chromatography, gas-chromatography / mass spectroscopic techniques, or by spectroscopical, rheological or mechanical tests.

Examples of such monitoring systems are found in Macromol. Symp. 2005, 230, 51-58 and in Kunststoffe International 5/2006. These publications describe in-line spectroscopy methods for monitoring extrusion processes. In these studies different spectroscopy methods were used to determinate the composition, filler dispersion and/or morphology of polymers or polymer blends while extruding.

Additional examples of monitoring systems for moving sheet-like materials are found in DE 199 19 801 A1, DE 41 34 590 A1, DE 101 57 792 A1, DE 10 2005 054 825 A1, DE 199 63 183 A1, and DE 10 2005 037 496 A1.

In recent years the idea of combinatorial compounding has received considerable attraction. In this approach a high number of specimens is formed during the production process, for example films with a local variation of chemical composition. Those parts of the film with the same chemical composition constitute single specimens of the film. The single specimens are investigated in a high thoughput screening process, for example via on-line testing methods. The test provides a selection between a variety of uninteresting specimens and a variety of interesting specimens. The latter are investigated in more detail by classical testing methods and the result thereof is another variety of ineligible specimens and a variety of eligible specimens. The latter are chosen for further product development.

For an efficient high throughput screening during a combinatorial compounding process or for an efficient screening of product properties during a production process improved on-line testing methods are needed.

It is an object of the present invention to provide an improved apparatus and an improved process of on-line monitoring of mechanical properties of filaments or films after compounding and extrusion. The apparatus detects mechanical properties on-line so that the process is not interrupted. Between processing, extrusion and testing no further process step is necessary, like cutting, punching or injection molding. The apparatus and process of this invention results in an acceleration of development time in combinatorial development processes and in a simple and reliable technique to monitor process parameters of filament and film production lines on-line.

Another object of the present invention is the provision of a simple and cost-efficient on-line monitoring technique for filament and film development and production lines.

The present invention relates to an apparatus for monitoring mechanical properties of a moving filamentary or planar body of solid material comprising:
a) means for causing a movement of the body along a predetermined passage, wherein in at least a part of the passage the body is levitating,
b) means for generating deflection and/or elongation of pre-determined length of the body which is positioned in that part of the passage, wherein the body is levitating, or which is positioned at the beginning or at the end of the predetermined passage, and
c) means for metering a force and/or a change of velocity created by the deflection and/or elongation of the body.

The apparatus of this invention can be used to monitor mechanical properties of any moving filamentary body or planar body of solid material. The specimens that can be investigated by the apparatus or method of this invention are charcterized by the presence of at least one dimension which is much smaller than the other dimension(s) of said specimen. Thus, besides filamentary specimens, planar specimens can be used.

Examples of filamentary specimens are filaments, yarns, threads or slivers.These filamentary specimens can be composed of different materials, for example of melt-spinnable polymers, of solution-spinnable polymers, of metal, or of glass or other fiber-forming inorganic materials.

Examples of planar specimens are films, sheet-like materials, such as textile sheets, paper sheets or sheets of inorganic fibrous materials or combinations thereof, such as laminates of different sheet-like materials. Films can be made from any film-forming material, such as from melt- or solution processable polymers films or metals or of combinations of different materials; textile sheets can be non-wovens, weavings, knittings or felts; paper sheets can be of different materials and forms, such as for example papers from cellulose fibers and/or man-made fibers or cardboard sheets; sheets of inorganinc fibrous materials can be glass fiber mats, mineral fiber mats or carbon fiber mats. These planar specimens can be composed of different materials, for example of different polymers, metals, glass or other film-forming inorganic materials or any combination thereof. Furthermore, laminates of different sheet-like materials can be used.

Means a) can be any device that is capable of causing a movement of the filamentary body or of the planar body along a predetermined passage thereby maintaining the body levitating for at least a part of the passage.

In a first or in a third embodiment means a) comprises a plurality of pairs of godet rolls, for example a first pair of two godet rolls and a second pair of two godet rolls, which are arranged in a fixed distance from each other. These pairs of godet rolls move the body between said rolls and cause the body to be levitating within the distance defined by the first and the second pair of godet rolls. Instead of two godet rolls a pair of godet rolls can consist of three or more godet rolls. Besides the pairs of godet rolls additional rolls can be present within the distance defined by the first and the second pair of godet rolls; these additional rolls can be used as guide rolls to control the pathway of movement of the body within the apparatus.

In an alternative second embodiment means a) comprises a plurality of pairs of godet rolls, for example a first pair of two godet rolls and a second pair of two godet rolls, which are movably arranged and have a varying distance from each other. These pairs of godet rolls move the body between said rolls and cause the body to be levitating within the distance defined by the first and the second pair of godet rolls. This second embodiment differs from the first embodiment as the pairs of godet rolls do not define a fixed distance from each other but said pairs rolls can be moved in relation to each other. In a preferred embodiment one pair of rolls is fixed at a position and a second pair of rolls can be moved in relation to the first pair of rolls. The movement of the second pair of rolls can be in the direction of the movement of the filamentary or planar body or can be in the reverse direction. But the movement of the second pair of rolls can be also transversal to the direction of the movement of the filamentary or planar body; or the movement of the second pair of rolls can generally be in angular direction with respect to the movement of the filamentary or planar body.

Instead of two godet rolls a pair of godet rolls can consist of three or more godet rolls. Besides the pairs of godet rolls additional rolls can be present within the distance defined by the first and the second pair of godet rolls; these additional rolls can be used as guide rolls to control the pathway of movement of the body within the apparatus.

Alternative means a) can be pairs of transportation belts.

Means b) can be any device that is capable of generating deflection and/or elongation of pre-determined length of the body.

The deflection and/or elongation of the filamentous or planar body can result in an elastic deformation of the body or in an inelastic deformation of the body or in an elastic and inelastic deformation of the body. Depending on the pathway of the body and/or the forces acting on the body during its pathway through means b) the quantity and manner of deformation can be controlled. Thus the body may be tested without any permanent deformation or the body may become deformed after its passage through means b).

In a first embodiment means b) is positioned in that part of the passage, wherein the body is levitating. A typical configuration of the apparatus of this embodiment is a combination of at least two pairs of godet rolls defining means a) which transport the body in a levitating manner said two pairs of godet rolls and a means b) positioned between the at least two pairs of godet rolls forming means a). Means b) in this embodiment forces the moving body to travel along a predetermined pathway causing the body to deform and/or to elongate in a controlled manner. Means b) in this first embodiment can be a plurality of rolls with profiled or flat surface including a plurality of interconnecting gearwheels or a combination of gearwheels and rolls through which the moving body is guided. The rolls and/or gearwheels constituting means b) are arranged in a manner that the pathway of the moving body is different from the normal pathway which the moving body would have taken if only means a) were present.

Thus the rolls and/or gearwheels constituting means b) can be arranged above, below or in the plane defined by the movement of the body between the rolls forming means a). The pathway of the body through the rolls and/or gearwheels constituting means b) can cause a predetermined deflection and/or elongation of said body; or the body can be guided in a straightforward manner through the rolls constituting means b) but the position of means b) in relation to means a) causes a pre-determined deflection and/or elongation of said body.

The rolls and/or gearwheels constituting means b) may be positioned at a fixed location in the apparatus or they may be moved in a predetermined manner during the operation. For example, a pair of rolls and/or gearwheels acting on a moving body between the godet rolls of means a) may be moved during the operation in a direction perpendicular to the movement of the body; this can create an additional deflection and/or elongation of the body during the measurement process, if the pair of rolls and/or gearwheels together with the moving body is moved in one direction; but a movement of the pair of rolls and/or gearwheels can also create a period of inactivity, if the single rolls and/or gearwheels are moved in opposite directions and are separated from the moving body. In this latter embodiment the moving body is tested in predetermined time intervals by the pair of rolls and/or gearwheels while in other predetermined time intervals there is no action between the rolls and/or gearwheels and the moving body.

In a second embodiment means b) is positioned at the beginning or at the end of the passage defined by means a). A typical configuration of the apparatus of this embodiment is that one part of means a) also constitutes means b). Thus at least one pair of godet rolls constituting a first portion of means a) is provided at a fixed location and at least another pair of godet rolls constituting a second portion of means a) is movable provided at another location. The combination of at least two pairs of godet rolls defining means a) act as a transportation means for the body. The movable second portion of means a) in addition constitutes means b). During operation of the apparatus means a) moves the body and in predetermined time intervals the position of the second portion of means a) is changed in a pre-determined manner. Any direction of movement of the second portion of means a) can be used, as long as this movement creates additional stress on the body. Thus the second portion of means a) can be moved for a predetermined length into the same direction as the movement of the body; this will create additional stress on the body. After the movement of the second portion of means a) is completed this second portion is moved back into the starting position. In an alternative manner the second portion of means a) can be moved for a predetermined length perpendicular or in an angular manner towards the direction of the movement of the body; this will create additional stress and deflection on the body. After the movement of the second portion of means a) is completed this second portion is moved back into the starting position. Means b) in this second embodiment forces the moving body during predetermined time intervals to travel along a predetermined pathway that differs from the normal pathway through means a) or the movement of means b) imparts additional stress on the body travelling the normal pathway through means a). Both modes of action cause the moving body to deform and/or to elongate in a controlled manner.

Means b) in this second embodiment can also be a plurality of rolls with profiled or flat surface including a plurality of interconnecting gearwheels or a combination of gearwheels and rolls through which the moving body is guided. Preferably a pair of at least two godet rolls is used defining in the beginning of a measurement cycle the normal pathway of the moving body through means a).

In a third embodiment means b) is positioned at the beginning or at the end of the passage defined by means a). A typical configuration of the apparatus of this embodiment is that one part of means a) also constitutes means b). Thus at least one pair of godet rolls constituting a first portion of means a) is provided at a fixed location and at least another pair of godet rolls constituting a second portion of means a) is provided at another fixed location. The combination of at least two pairs of godet rolls defining means a) act as a transportation means for the body. The second portion of means a) in addition constitutes means b). During operation of the apparatus means a) moves the body and in predetermined time intervals the rotational speed of the second portion of means a) is changed in a pre-determined manner or the second portion of means a) operates at a constant rotational speed that is higher than the rotational speed of the first portion of means a). Any change of rotational speed of the second portion of means a) or any proportion of the rotational speed of the first and the second portions of means a) can be used, as long as this change of rotational speed or this difference in rotational speed creates additional stress on the body moving between the first and the second portion of means a). Thus the second portion of means a) can be faster rotated in a predetermined manner compared to the first portion of means a) and will cause transportation and additional stress on the body.

In cases where different rotational speeds of the second portion of means a) are used the rotational speed of the second portion of means a) will receive its final value at the end of a measurement cycle. At this time the rotational speed of the second portion of means a) is set to the starting value or to any other predetermined value to start another measurement cycle.

Means b) in this third embodiment forces the moving body during predetermined time intervals to travel along a predetermined speed that differs from the speed at the beginning of the measurement cycle, or means b) in this third embodiment forces the moving body during the measurement cycle to travel along a fixed speed but creating a tension between the first and the second portions of means a). These modes of action cause the moving body to deform and/or to elongate in a controlled manner.

Means b) in this third embodiment can also be a plurality of rolls with profiled or flat surface including a plurality of interconnecting gearwheels or a combination of gearwheels and rolls through which the moving body is guided. Preferably a pair of at least two godet rolls is used defining in the beginning of a measurement cycle the normal pathway of the moving body through means a).

The pathway of the body defined by the first pair of rolls and/or gearwheels of means a) and defined by the second pair of rolls and/or gearwheels of means a), in addition constituting means b) causes a predetermined deflection and/or elongation and/or change of velocity of said body during the movement of the second pair of rolls and/or gearwheels.

The second pair of rolls and/or gearwheels constituting means b) of the second embodiment are moved in a predetermined manner during the operation. After a measurement cycle has been completed the second pair of rolls and/or gearwheels is moved back to the starting position. In this second embodiment the moving body is tested in predetermined time intervals by the movement of the second pair of rolls and/or gearwheels while in other predetermined time intervals there is no testing activity when the second pair of rolls and/or gearwheels is in the starting position and acts only as portion of means a).

Means c) can be any device that is capable of metering a force and/or a change of velocity of the moving body that is created by the deflection and/or elongation of the body caused by the action of means b).

Typically a force sensor is coupled with an axis of a roll forming a part of means b). If an deformation and/or an elongation of the moving body is caused by the action of means b) there is acting a force on said axis of the roll which is detected by said force sensor.

In an alternative embodiment a sensor for monitoring the velocity of a roll forming a part of means b) is present. If an deformation and/or an elongation of the moving body is caused by the action of means b) there is a change of velocity of said roll which is detected by said velocity sensor.

Any force sensors or velocity sensors can be used. Examples of force sensors are sensors based on strain gauge techniques or sensors integrated in one or more bearings of rolls. Examples of velocity sensors are incremental encoders with different solutions.

Preferably a force sensor is used which is connected to an axis of a roll of means b). But other embodiments are also possible, provided these allow the determination of the force created by the deformation and/or elongation of the moving body.

The change of force or of velocity created by the deformation and/or elongation of the moving body is used to monitor selected properties of the body under investigation.

In a film or filament formation or processing procedure different variables can influence the properties of the final product.

Besides the selection of the material (combination) process variables or other process factors can influence the final product. Examples of process variables are haul-off speed of chill rolls, temperature of chill-rolls, take-up roll speed, film or filament guide way, rotation speed of melt pumps, die temperature, or die geometry. Furthermore, process factors, such as film or filament thickness or film or filament morphology, may be of influence for the properties final product.

Furthermore, the action of means b) may influence the measurement. For example, change of the distance of a pair of interconnecting gearwheels from each other may result in a different elongation and/or velocity of deformation of the film or filament moving inbetween said gearwheels. Depending on the amount of interconnection between the gears there may be caused an elastic deformation of the film or filament or an elastic and inelastic deformation thereof.

As an indicator for the amount deflection and/or elongation and/or deformation velocity of the moving film or filament the force acting upon a load cell may be determined.

In a preferred embodiment of this invention the apparatus in addition contains a means for calculating a mechanical property d) using the length of deflection and/or elongation and/or deformation velocity of the body and the force created by said deflection and/or elongation and/or velocity. Besides the force determined by a force sensor here the length of deflection and/or elongation and/or deformation velocity must be determined. This can be performed by calculating or by measuring the pathway of the moving body created by the action of means b) compared to the pathway of the moving body in the absence of means b).

In another preferred embodiment of the apparatus of this invention means a) is formed by at least two pairs of godet rolls arranged at fixed positions and defining the predetermined passage of the body, wherein between said pairs of godet rolls the body is levitating and wherein at least one godet roll is driven by a motor.

In still another preferred embodiment of the apparatus of this invention means b) is a pair of interlocking gearwheels defining a passage for the body between said gearwheels which pair of gearwheels is positioned in the part of the passage, where the body is levitating.

In still another preferred embodiment of the apparatus of this invention means c) is a force sensor measuring the force created by the deflection and/or elongation of the body during the passage through the interlocked gearwheels.

In a very preferred variant the apparatus of this invention contains a pair of interlocking gearwheels as means b), wherein at least one of the gearwheels is movable in a direction perpendicular to the direction of movement of the body and wherein the pair of gearwheels is arranged in a first and in a second position, the first position defining a measuring position with interlocking gearwheels and the second position defining an inactive position with at least one of the gearwheels not being in contact with the body.

In another preferred variant the apparatus of this invention has a means b) which is a pair of rolls arranged above or below the plane of movement of the body between means a), said pair of rolls defines a passage between said rolls for the body which pair of rolls is positioned in the part of the passage, where the body is levitating.

In this variant means c) is preferably a force sensor measuring the force created by the deflection and/or elongation of the body during the passage through the pair of rolls.

In a preferred version of this variant at least one of the rolls is movable in a direction perpendicular to the direction of movement of the body and the pair of rolls is arranged in a first and in a second position, the first position defining a measuring position with the pair of rolls contacting each other and the second position defining an inactive position with at least one of the rolls not being in contact with the body.

In another preferred variant the apparatus of this invention contains a means a) which is formed by at least two pairs of godet rolls, one pair of godet rolls being arranged at a fixed position and a second pair of godet rolls being movable arranged to define a predetermined passage of variable length for the body, wherein said second pair of godet rolls concurrently define means b) and wherein said second pair of godet rolls is connected to a force sensor measuring the force created by the deflection and/or elongation of the body during the movement of the second pair of godet rolls.

Preferably in the apparatus of this invention a filament or yarn or a plurality of filaments or yarns, preferably made of thermoplastic spinnable polymers, is used as a moving body.

Preferably in the apparatus of this invention a polymer film, a metal film, a textile sheet material or a sheet of inorganic fibrous material is used as a moving body. The apparatus of this invention may be easily incorporated into a production line for a filamentary or planar body of solid material.

The invention therefore also relates to a production line for a filamentary or planar body of solid material comprising an apparatus for monitoring mechanical properties of the body as defined above, wherein the apparatus is arranged close to the production line and wherein the body of solid material transferred to the apparatus is a portion from the production line which is bypassed from the production line to the apparatus, or wherein the apparatus is integrated into the production line and takes a first and a second position, the first position defining a measuring position and the second position defining an inactive position, wherein the first and the second position are captured during predetermined time intervals.

The invention furthermore relates to a process for monitoring mechanical properties of a moving filamentary or planar body of solid material comprising the steps:
i) moving the body along a predetermined passage using means a) for causing a movement of the body, while keeping the body levitating during at least a part of the passage,
ii) generating deflection and/or elongation of predetermined length in the body by using a means b), which is positioned in the part of the passage, wherein the body is levitating, or which is positioned at the beginning or at the end of the predetermined passage, and
iii) metering the force and/or the change of velocity created by the deflection and/or the elongation of the body by using a means c).

In a preferred variant of the process of this invention a mechanical property of the body is calculated by using the length of deflection and/or elongation of the body and the force created by said deflection and/or elongation.

The invention is now explained in more detail with reference to the attached drawings.
Figure 1 is a schematic representation of one embodiment of the apparatus of this invention using a pair of interconnecting gearwheels arranged at a fixed position between two pairs of godet rolls.
Figure 2 is a schematic representation of another embodiment of the apparatus of this invention using two packets of godet rolls arranged in a movable position to each other.

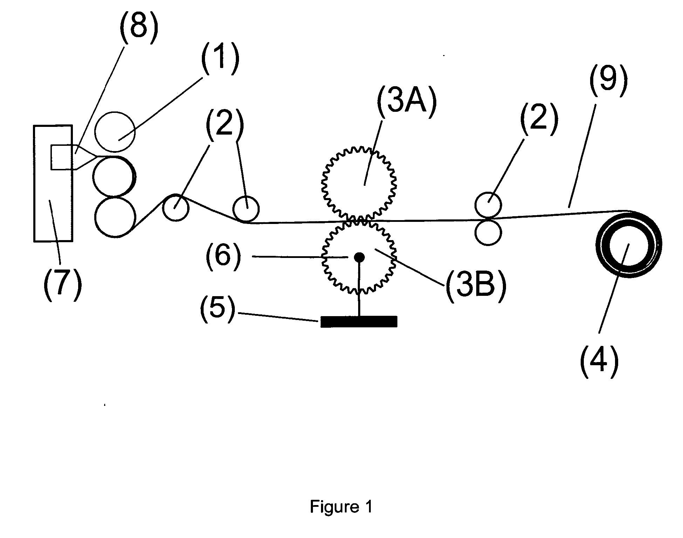

Figure 1 shows a melt pump (7) for transporting a polymer melt through a die (8) to form a film (9). This is transported by the action of a pair of chill rolls (1) and a take-up roll (4). Chill rolls (1) and take-up roll (4) form means a). At least one of the chill rolls (1) and the take-up roll (4) are actively moved by means of motors. Besides these rolls additional guide rolls (2) are present. Film (9) is levitating between chill rolls (1) and take-up roll (4). In the passage of film (9) between chill rolls (1) and take-up roll (4) a pair of interconnecting gearwheels (3a, 3b) is positioned forming means b). Film (9) or at least a part thereof is forced to move between said gearwheels. These cause a deformation of the film during its passage through the gearwheels (3a, 3b). Axis (6) of the lower gearwheel (3b) is connected to a load cell (5) forming means c) which determines the force exerted by the film during its passage through the gearwheels (3a, 3b). In a preferred embodiment the gearwheels (3a, 3b) can be moved perpendicular to the direction of the movement of film (9) from a measurement position into a hold position and vice versa. The load exerted by axis (6) on load cell (5) is monitored as a function of time and in dependance of process variables of the film forming process, such as temperature of selected rolls, die temperature or film composition.

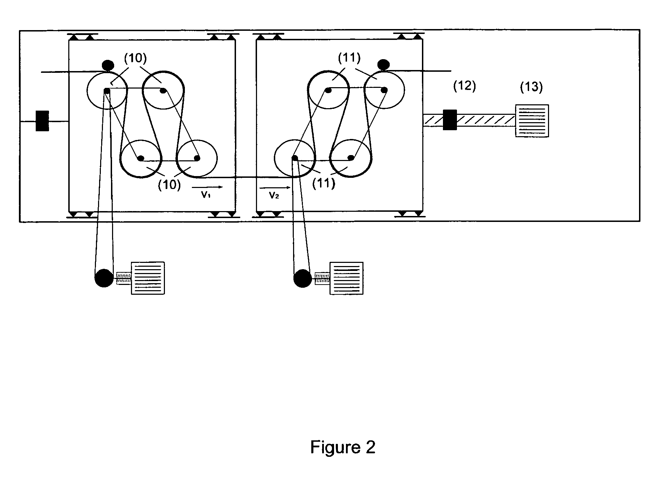

Figure 2 shows a film (9) that is transported via a first pluarality of godet rolls (10) and a second plurality of godet rolls (11). At least one roll in each plurality of godet rolls (10,11) is actively moved by a motor. First and second plurality of godet rolls (10, 11) form means a). The first plurality of godet rolls (10) is located at a fixed position whereas the second plurality of godet rolls (11) is movable in the direction of the movement of film (9) or against the direction of the movement of film (9). The second plurality of godet rolls (11) also forms means b). The second plurality of godet rolls (11) is connected via a force sensor (12), forming means c), to a motor (13) which moves said second plurality of godet rolls (11) in the direction of the movement of the film (9) or into the opposite direction. At the beginning of a measurement cycle film (9) is moved by the action of the first and second pair of godet rolls (10,11) and in addition the second pair of godet rolls (11) is moved in the direction of the movement of film (9). This creates an additional stress on the film (9) (besides the stress created by the movement of the single rolls of the first and second pair of godet rolls) resulting in an additional elongation thereof and the force exerted by this additional stress is monitored via force sensor (12). At the end of the measurement operation the movement of the second pair of godet rolls (11) is stopped and this is moved contrary to the direction of movement of film (9) into the starting position from where the measurement cycle can be started again.

## Claims

1. An apparatus for monitoring mechanical properties of a moving filamentary or planar body of solid material comprising:
a) means for causing a movement of the body along a predetermined passage, wherein in at least a part of the passage the body is levitating,
b) means for generating deflection and/or elongation of pre-determined length of the body which is positioned in that part of the passage, wherein the body is levitating, or which is positioned at the beginning or at the end of the predetermined passage, and
c) means for metering a force and/or a change of velocity of the moving body created by the deflection and/or elongation of the body.

2. The apparatus of claim 1, wherein the apparatus in addition contains a means of calculating a mechanical property d) using the length of deflection and/or elongation of the body and the force created by said deflection and/or elongation.

3. The apparatus of claim 1, wherein means a) is formed by at least two pairs of godet rolls arranged at fixed positions and defining the predetermined passage of the body, wherein between said pairs of godet rolls the body is levitating and wherein at least one godet roll is driven by a motor.

4. The apparatus of any one of claims 1 to 3, wherein means b) is a pair of interlocking gearwheels defining a passage for the body between said gearwheels which pair of gearwheels is positioned in the part of the passage, where the body is levitating.

5. The apparatus of claim 4, wherein means c) is a force sensor measuring the force created by the deflection and/or elongation of the body during the passage through the interlocked gearwheels.

6. The apparatus of any one of claims 4 or 5, wherein at least one of the gearwheels is movable in a direction perpendicular to the direction of movement of the body and wherein the pair of gearwheels is arranged in a first and in a second position, the first position defining a measuring position with interlocking gearwheels and the second position defining an inactive position with at least one of the gearwheels not being in contact with the body.

7. The apparatus of any one of claims 1 to 3, wherein means b) is a pair of rolls arranged above or below the plane of movement of the body between means a), said pair of rolls defines a passage between said rolls for the body which pair of rolls is positioned in the part of the passage, where the body is levitating.

8. The apparatus of claim 7, wherein means c) is a force sensor measuring the force created by the deflection and/or elongation of the body during the passage through the pair of rolls.

9. The apparatus of any one of claims 7 or 8, wherein at least one of the rolls is movable in a direction perpendicular to the direction of movement of the body and wherein the pair of rolls is arranged in a first and in a second position, the first position defining a measuring position with the pair of rolls contacting each other and the second position defining an inactive position with at least one of the rolls not being in contact with the body.

10. The apparatus of any one of claims 1 to 2, wherein means a) is formed by at least two pairs of godet rolls, one pair of godet rolls being arranged at a fixed position and a second pair of godet rolls being movable arranged to define a predetermined passage of variable length for the body, wherein said second pair of godet rolls concurrently define means b) and wherein said second pair of godet rolls is connected to a force sensor measuring the force created by the deflection and/or elongation of the body during the movement of the second pair of godet rolls.

11. The apparatus of any one of claims 1 to 2, wherein means b) is positioned at the beginning or at the end of the passage defined by means a), wherein at least one pair of godet rolls constituting a first portion of means a) is provided at a fixed location and at least another pair of godet rolls constituting a second portion of means a) and constituting means b) is provided at another fixed location and wherein in predetermined time intervals the rotational speed of the second portion of means a) is changed in a pre-determined manner or wherein the second portion of means a) operates at a constant rotational speed that is higher than the rotational speed of the first portion of means a) which change of rotational speed or which difference in rotational speed creates additional stress on the body moving between the first and the second portion of means a).

12. The apparatus of any one of the preceeding claims, wherein the body is a filament or yarn or a plurality of filaments or yarns, preferably made of thermoplastic spinnable polymers.

13. The apparatus of any of claims 1 to 11, wherein the body is a polymer film, a metal film, a textile sheet material, a paper sheet material, or a sheet of inorganic fibrous material.

14. A production line for a filamentary or planar body of solid material comprising an apparatus for monitoring mechanical properties of the body according to any of the preceeding claims, wherein the apparatus is arranged close to the production line and wherein the body of solid material transferred to the apparatus is a portion from the production line which is bypassed from the production line to the apparatus, or wherein the apparatus is integrated into the production line and takes a first and a second position, the first position defining a measuring position and the second position defining an inactive position, wherein the first and the second position are captured during predetermined time intervals.

15. A process for monitoring mechanical properties of a moving filamentary or planar body of solid material comprising the steps:
i) moving the body along a predetermined passage using means a) for causing a movement of the body, while keeping the body levitating during at least a part of the passage,
ii) generating deflection and/or elongation of predetermined length in the body by using a means b), which is positioned in the part of the passage, wherein the body is levitating, or which is positioned at the beginning or at the end of the predetermined passage, and
iii) metering the force created by the deflection and/or the elongation of the body by using a means c).

16. The process of claim 15, wherein the mechanical property of the body is calculated by using the length of deflection and/or elongation of the body and the force created by said deflection and/or elongation.
